# EUROPÄISCHE PATENTSCHRIFT

(11) **EP 0 645 122 B1**
(45) Veröffentlichungstag und Bekanntmachung des Hinweises auf die Patenterteilung: **03.12.1997**
(21) Anmeldenummer: 94113486.8
(22) Anmeldetag: 30.08.1994
(51) Int. Cl.: A61C 5/06, A61M 5/315

(54) **Spritze zum dosierten Abgeben von viskosen Werkstoffen, insbesondere von dentalen Werkstoffen**
Syringe for dosing viscous substances, in particular for dental substances
Seringue de dosage de substances visqueuses, en particulier de substances dentaires

(30) Priorität: 23.09.1993 DE 4332308
(43) Veröffentlichungstag der Anmeldung: 29.03.1995
(73) Patentinhaber: HERAEUS KULZER GMBH, D-61273 Wehrheim (DE)
(72) Erfinder: Eykmann, Rudolf, D-61273 Wehrheim (DE); Fritze, Joachim, Dr., D-61381 Friedrichsdorf (DE); Uhrig, Birgit, D-61276 Neu-Anspach (DE); Schödel, Dieter, Dr., D-65193 Wiesbaden (DE)
(74) Vertreter: Grimm, Ekkehard, Dipl.-Phys.

(56) Entgegenhaltungen:
- DE-A- 3 930 817
- DE-A- 4 200 044
- DE-B- 2 644 930

## Beschreibung

Die vorliegende Erfindung betrifft eine Spritze zum dosierten Abgeben von pastösen Werkstoffen mit einer den Werkstoff aufnehmenden Patrone, in die von deren einem Ende aus ein Drehkolben eintaucht, wobei der Drehkolben an seinem Eintauchende gegen einen Stopfen, der ein scheibenförmiges Flächenteil aufweist, das den Innenquerschnitt der Patrone dichtend verschließt, anliegt.

Eine solche Spritze ist beispielsweise aus der Produktinformation der Heraeus Kulzer GmbH "Charisma-Inlays - Gewinn durch Perfektion und Ästhetik" (31292/D 125 sK dt./WPR 12 12 200) oder aus der DE-A-4 200 044 bekannt. Diese Spritzen, in denen unter dem Produktnamen "Charisma" (Charisma ist eingetragenes Warenzeichen der Heraeus Kulzer GmbH) vertriebene, viskose Dentalwerkstoffe abgefüllt werden, weisen eine Patrone auf, in die der Dentalwerkstoff eingefüllt wird. Diese Patrone ist an ihrem Austragsende im Außenumfang in Form einer Material-Austrittsdüse verjüngt ausgebildet, auf die eine Verschlußkappe aufgesetzt wird. In das dem Austragsende gegenüberliegende Ende der Patrone taucht ein Drehkolben ein, der sich an seinem Ende in einen relativ steifen, hülsenförmigen Stopfen einlegt, der seinerseits gegen den in der Patrone abgefüllten viskosen Werkstoff anliegt. Der Drehkolben ist mit einem Gewinde versehen, das in einem Lager in Form einer Mutter geführt ist. Um die Dentalwerkstoffe aus der Patrone auszutragen, wird die Verschlußkappe der Patrone entfernt und der Drehkolben, der an seinem Ende ein Griffteil besitzt, in die Patrone hineingedreht, so daß der Stopfen zum Austragsende der Patrone hin verschoben wird und den Werkstoff unter Druck setzt. Diese Spritzen haben sich im Einsatz über Jahre gut bewährt.

Die angegebenen Patronen, die Dentalmaterialien aufnehmen, werden üblicherweise von ihrem hinteren Ende, d.h. dem Ende aus, das der Austrittsdüse gegenüberliegt, befüllt. Nach dem Befüllen von hinten wird dann der eingangs beschriebene hülsenförmige Verschlußstopfen in das offene Befüllende der Patrone eingedrückt, bis er gegen das eingefüllte Material anliegt, gegen den dann später der Drehkolben zum Austragen des Materials drückt. Da solche Patronen, die das Dentalmaterial aufnehmen, auf der Innenseite eine geringe Konizität zu dem Ende hin aufweisen, von dem aus das Dentalmaterial eingefüllt und der Stopfen eingesetzt wird, die zum Entformen des Werkstücks nach dem Spritzguß notwendig ist, liegt der Stopfen mit unterschiedlicher Spannung in axialer Richtung der Patrone an deren Innenwand an. Gerade bei niedrig viskosen Materialien kann die Dichtigkeit der Patrone zu Problemen führen. Falls ein Stopfen verwendet wird, der eine höhere Dichtigkeit am Übergang zu der Innenwand der Patrone aufweist, entsteht beim Füllvorgang der Patrone mit Material nach Einsetzen des Stopfens zwischen Material und Stopfen ein Luftpolster, das nicht entweichen kann. Um diesem Problem zu entgegnen, wurden solche Stopfen mit einem mittigen Loch versehen. Der Drehkolben ist mit einem Dorn versehen, um in das Loch einzuschnappen und dieses zu verschließen. Falls bei einer solchen Ausführungsform nach einem Austrag von Material aus der Patrone der Stopfen und damit das Material entlastet werden soll, kann sich mit einem zu weiten Zurückdrehen des Drehkolbens aus der Spritze der Dorn aus dem Loch des Stopfens herausziehen, so daß das in der Patrone befindliche Material der Umgebungsluft ausgesetzt wird. Hierbei kann es zu einer Aushärtung des Materials kommen, zumindest zu einer Änderung der Materialeigenschaften. Eine wesentliche Anforderung an solche Spritzen ist diejenige, daß das Material genau dosiert werden kann, das für die zu behandelnde oder zu bearbeitende Stelle an einem Zahn oder einem Zahnersatz verwendet wird. Darüberhinaus wird eine solche Spritze nicht in einem Arbeitsvorgang entleert, sondern sie wird zwischengelagert und das darin befindliche Material von Anwendung zu Anwendung ausgetragen. Wesentlich hierbei ist, daß nach einem Austrag des Materials aus der Austrittsöffnung oder Austrittsdüse der Spritze kein Material nachläuft, um so zu verhindern, daß einerseits die genau dosierte Menge ausgetragen wird und andererseits vor dem Wiederverschluß der Austrittsdüse mit einer Verschlußkappe kein Material an den Flächen, an denen die Verschlußkappe anliegt, klebt. Aus vorstehendem Grund ist man dazu übergegangen, den Drehkolben mit dem Stopfen zu verbinden, so daß der Drehkolben beim Zurückdrehen entgegen der Austragsrichtung den Stopfen zurückzieht und damit das Material entlastet bzw. ein Unterdruck in der Patrone erzeugt wird, so daß kein weiteres Material aus der Austragsdüse austritt. Diese Anordnung führt aber dazu, daß bei sehr dicht an der Innenwand der Patrone anliegendem Stopfen das Material sehr weit in die Patrone zurückgezogen wird, so daß der Drehkolben für einen erneuten Austrag des Materials aus der Patrone sehr lange gedreht werden muß. Weiterhin ist der Drehkolben praktisch ein fester Bestandteil des Stopfens, so daß nach einem Gebrauch einer solchen Spritze der Drehkolben mit dem Stopfen nicht wiederverwendbar ist.

Ausgehend von dem vorstehend beschriebenen Stand der Technik liegt der vorliegenden Erfindung die Aufgabe zugrunde, eine Spritze zum dosierten Abgeben von pastösen, insbesondere dentalen Werkstoffen der eingangs beschriebenen Art anzugeben, die ein gezieltes Entlasten des in der Patrone eingefüllten Materials bei Beendigung eines Austrags vornimmt und mit der über die gesamte Länge der Patrone eine gute Abdichtung des Materials zur Außenseite hin durch den Stopfen, d.h. zur Seite des Drehkolbens hin, erzielt wird.

Die vorstehende Aufgabe wird bei einer gattungsgemäßen Spritze dadurch gelöst, daß das scheibenförmige Flächenteil des Stopfen mindestens teilweise als Membranfläche ausgebildet ist, die im entlasteten Zustand eine stabile Lage bildend gegen die Eintauchrichtung des Drehkolbens so vorgespannt ist, daß sie nach einer Verschiebung durch den Drehkolben im entlasteten Zustand in diese stabile Lage reversibel zurückkehrt, und daß die Membranfläche aus Kunststoff gebildet ist. Durch die Membranfläche, die genau durch eine geeignete Form, Dicke und Vorspannung eingestellt werden kann, ist es möglich, eine gezielte Entlastung des dentalen Materials beim Zurückziehen des Drehkolbens aus der Patrone zu erreichen. Die Membranfläche ist in einer Ausführungsform so gestaltet, daß sie nach dem Einsetzen in die Patrone zu dem Drehkolben hin gewölbt bzw. vorgespannt ist. Gegen diese Wölbung drückt beim Eindrehen des Drehkolbens in die Patrone das Ende des Drehkolbens und verformt die Membranfläche in die entgegengesetzte Richtung zu dem Material hin. Nach einem weiteren Verschieben des Drehkolbens wird dosiert eine portionierte Menge Material aus der Austragsöffnung der Patrone ausgetragen. Danach wird die Drehung des Drehkolbens umgekehrt, so daß sie sich wieder aus der Patrone zurückzieht. Zwischen dem Ende des Drehkolbens und der Membranfläche des Stopfens entsteht ein freier Zwischenraum, der ermöglicht, daß die Membranfläche wieder in ihre vorgespannte Ausgangsstellung zurückkehrt, in der die Wölbung zu dem Ende des Drehkolbens hin gerichtet ist.

Bevorzugt wird der Stopfen becherförmig und dessen Boden als elastisch verformbare Fläche ausgebildet. Der Boden oder die Membranfläche sind in dieser Ausgestaltung an einer Art Ringteil geführt, das eine gute Anlagefläche und Führung an der Innenwand der Patrone bildet. Ein solcher ringförmiger Rand besitzt eine ausreichende Stabilität, um die Membranfläche daran zu lagern.

In einer einfachen Ausgestaltung wird, wie bereits erwähnt, die Vorspannung der Membranfläche durch eine Wölbung der Membranfläche gegen die Eintauchrichtung des Drehkolbens erzielt. Die Vorspannung kann in dieser Ausführung des Stopfens durch die Stärke der Wölbung, eine unterschiedliche Dicke der Membranfläche von der Mitte zur Außenseite hin - in der Mitte sollte die Membranfläche dünner sein als an ihrem äußeren Rand - und durch die Elastizität des Materials, aus dem der Stopfen bzw. die Membranfläche hergestellt wird, eingestellt werden.

Eine besonders gute Dichtwirkung zwischen der Innenwand der Patrone und dem Stopfen wird durch eine umlaufende Dichtlippe erreicht, die in einer bevorzugten Ausführung über die Bodenfläche nach unten, d.h. zum Austragsende der Patrone hin, und über die Seitenwände nach außen vorsteht. Weiterhin wird, um die Elastizität der Membranfläche bis zu ihrem äußeren Rand hin zu gewährleisten, auf der Innenseite des becherförmigen Stopfens im Übergangsbereich zwischen der Becherwand und dem Becherboden eine zumindest teilweise umlaufende Nut ausgebildet. Durch diese Nut wird eine Sollbiegestelle der Membranfläche im Übergangsbereich zwischen Membran und Seitenwand des becherförmigen Stopfens gebildet. Außerdem wird durch diese bevorzugt vollständig umlaufende Nut ein ausreichender Freiraum gebildet, der weitgehend beim Verschieben der Membranfläche in ihre stabile, vorgespannte Lage geschlossen wird. Die umlaufende Dichtlippe, die nach unten über den Boden des becherförmigen Stopfens und nach außen über die Seitenwand des becherförmigen Stopfens vorsteht, kann, durch Ausbildung in einer entsprechenden Länge, so zur Achse der Patrone hin infolge ihrer Anlage an der Patronenwand gebogen werden, daß ein Biegemoment auf die Membranfläche hervorgerufen wird, so daß sich die Membranfläche nach innen zu dem Ende des Drehkolbens hin vorspannt. Eine solche Ausbildung des Stopfens hat insbesondere auch den Vorteil, daß die Vorspannung der Membranfläche erst beim Einsetzen des Stopfens in die Patrone hervorgerufen wird und auch nach einer längeren Lagerung der Stopfen im Anschluß an ihre Herstellung und vor dem Einbau in eine Spritze eine ausreichende Vorspannung der Stopfen bzw. deren Membranflächen entsteht.

Neben der einen, über den Becherboden vorstehenden Dichtlippe können weitere Rillen und/oder Nuten in der Außenseite der Seitenwände des becherförmigen Stopfens gebildet werden, wobei die verbleibenden Stege zwischen den Rillen einzelne Dicht- und Führungsbereiche bilden.

Der Stopfen selbst bzw. dessen Seitenwände können konisch ausgebildet sein, wobei sich der Durchmesser der Seitenwände zu deren freiem Ende hin vergrößert. In einer solchen Ausbildung des Stopfens wird am hinteren Bereich des Stopfens durch den vergrößerten Durchmesser eine weitere Führungs- und Anlagefläche, die dicht an der Innenwand der Patrone anliegt, erzielt.

Der Stopfen kann in die Patrone so eingesetzt sein, daß er nach dem Befüllen der Patrone diese auf der einen Seite dichtend abschließt, so daß nach der Befüllung keine weiteren Verschlußkappen, auch nicht im Hinblick auf eine Zwischenlagerung der Patrone, auf dieses Ende der Patrone aufgesetzt werden müssen.

Der Drehkolben sollte an seinem Ende gewölbt verlaufen, und zwar bevorzugt in einer solchen Abrundung des Kolbenendes, die der maximalen Auslenkung der Bodenfläche des Stopfens in Vorschubrichtung des Drehkolbens entspricht. Selbst bei hoch viskosen Materialien tritt keine Beschädigung der Membranfläche auf, da sie sich auch unter einer großen aufgebrachten Kraft an die Wölbung des Endes des Drehkolbens anlegt.

In einer weiteren bevorzugten Ausbildung der Spritze weist die Patrone auf ihrer Innenseite, und zwar an dem Ende, an dem der Drehkolben in die Patrone eintaucht, einen Anschlag auf, gegen den sich der Stopfen beim Befüllen vom Austragsende aus anlegen kann. Dieser Anschlag kann z.B. durch eine stufenförmige Verringerung des Innendurchmessers der Patrone gebildet sein.

Die Membranfläche des Stopfens sollte eine Dicke zwischen 0,2 und 0,6 mm Aufweisen, wobei eine Dicke von etwa 0,4 mm in der Mitte der Membranfläche als bevorzugt anzusehen ist. Die nach unten über die Bodenfläche des Stopfens vorstehende umlaufende Dichtlippe, durch die das angegebene Moment auf die Membranfläche ausgeübt wird, sollte etwa 0,5 bis 1 mm über die Bodenfläche vorstehen. Die Ausnehmung bzw. der Radius, der auf der Innenseite im Übergangsbereich zwischen Bodenfläche und Seitenwand des Stopfens ausgebildet ist, sollte etwa 0,5 mm betragen, mit einer Tiefe von etwa 0,2 bis 0,3 mm.

Weitere Einzelheiten und Merkmale der Erfindung ergeben sich aus der nachfolgenden Beschreibung eines Ausführungsbeispiels anhand der Zeichnung. In der Zeichnung zeigt:
- Figur 1: eine seitliche Draufsicht auf eine Spritze mit einem Adapter und einer darin eingesetzten Patrone,
- Figur 2: eine Schnittdarstellung des Stopfens der Spritze nach Figur 1 entlang der Achse der Patrone bzw. der Achse des Stopfens, und
- Figur 3: einen Schnitt durch die Patrone der Spritze nach Figur 1 mit zwei unterschiedlichen Stellungen des Stopfens in der Patrone, und zwar zum einen in einer zum vorderen Austragsende verschobenen und zum anderen in einer hinteren, zurückgezogenen Stellung.

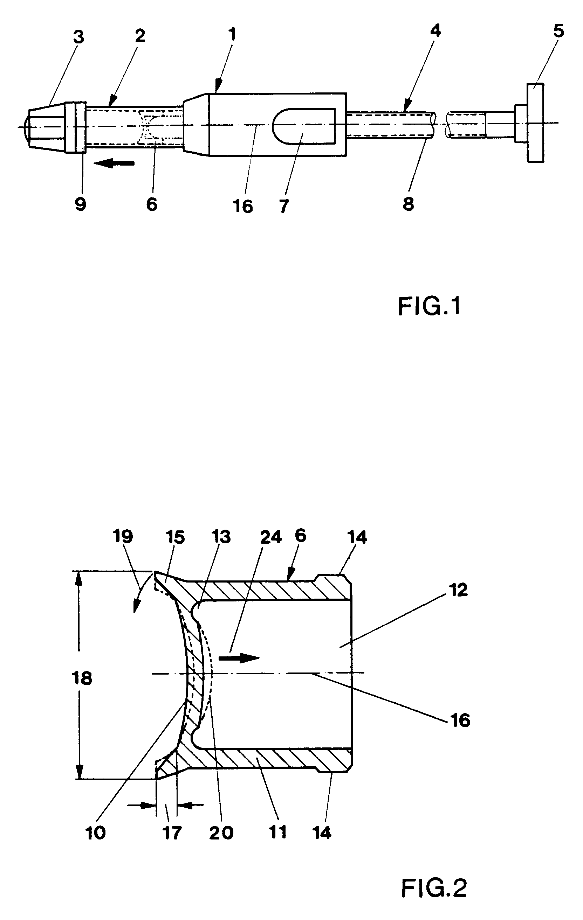

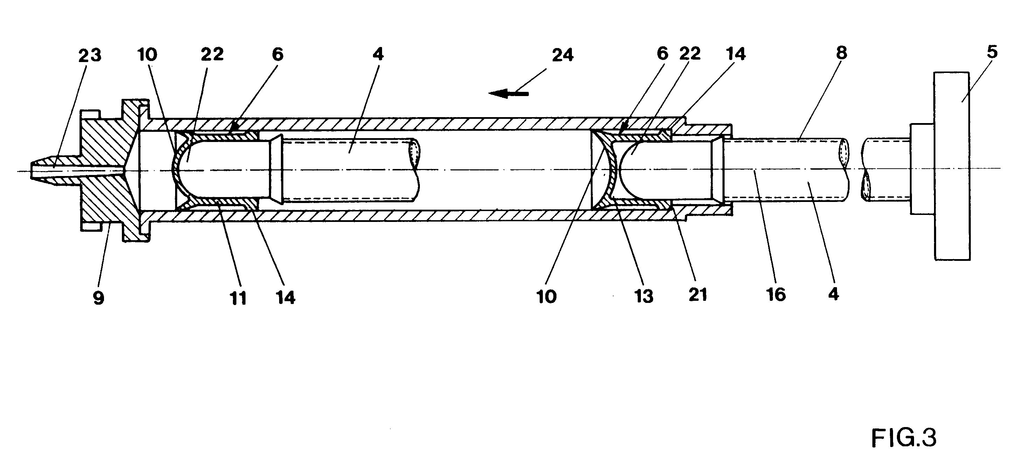

Eine Spritze zum dosierten Austragen von viskosen Werkstoffen, wie sie in Figur 1 dargestellt ist, weist einen Adapter 1 auf, in dem eine langgestreckte Patrone 2 gehalten ist, die an ihrem freien, aus dem Adapter 1 vorstehenden Ende mit einer Verschlußkappe 3 verschlossen ist. Die Patrone 2 ist mit einem auszutragenden, zu verarbeitenden Werkstoff befüllt. In den Adapter 1 tritt auf dem der Patrone 2 gegenüberliegenden Ende ein Gewinde oder Drehkolben 4 ein, der einen Griff 5 an seinem freien Ende besitzt. Das Eintauchende des Drehkolbens 4 drückt gegen einen Stopfen 6 in der Patrone 2, der in Figur 1 mit unterbrochenen Linien angedeutet ist. Der Drehkolben 4 wird in dem Adapterteil 1 in einem Lager 7 gehalten, in dem er mit seinem Gewinde 8 geführt ist. Um Material aus der Patrone 2 über die Austrittsdüse 9, wie sie in verschiedenen Ausführungen in den Figuren 2 und 3 gezeigt ist, auszutragen, wird der Drehkolben 4 am Griff 5 verdreht, so daß der Stopfen 6 in Richtung des Pfeils in Figur 1 vorgeschoben wird und gegen das in der Patrone 2 eingefüllte Material drückt. Nach dem Gebrauch wird der Stopfen 6 entlastet, indem der Drehkolben 4 zurückgedreht wird bzw. die Verbindung zwischen Drehkolben 4 und dem Stopfen 6 gelöst wird, und anschließend wird die Verschlußkappe 3 auf die Austrittsdüse 9 aufgesetzt.

Der Stopfen 6, wie er in Figur 2 in einer vergrößerten Darstellung zu sehen ist, ist in Form eines becherförmigen Teils ausgebildet mit einem Becherboden 10, der eine Membranfläche bildet, und Seitenwänden 11. Der Becherboden bzw. die Membranfläche 10 ist zu der offenen Seite 12 des Stopfens 6 hin vorgespannt bzw. konkav gewölbt. Der Radius der Wölbung der Membranfläche 10 des Stopfens 6 in der stabilen Lage beträgt bei der in Figur 2 dargestellten, nicht in der Patrone 2 eingesetzten Stellung etwa 100 bis 150 mm. Im Übergangsbereich zwischen Becherboden oder Membranfläche 10 und der Seitenwand 11 ist eine umlaufende Nut 13 gebildet mit einem Radius von etwa 0,5 mm und einer Tiefe, von der Innenseite der Membranfläche 10 ausgehend, von etwa 0,2 mm. An dem oberen Ende der Seitenwände 11 läuft auf der Außenseite ein Steg 14 um, der eine Anlage- oder Führungsfläche an der Innenwand der Patrone 2 bildet, wie dies in Figur 3 zu sehen ist. Anstelle dieses Steges kann auch an diesem Ende der Außendurchmesser des Stopfens 6 vergrößert sein, so daß die Seitenwände 11 konisch verlaufen. An dem anderen Ende der Seitenwände 11 steht eine umlaufende Dichtlippe 15 sowohl, in Richtung der Achse 16 des Stopfens gesehen, über die Unterseite der Membranfläche 10 als auch über die Seitenwände 11 radial nach außen vor. Der radiale Überstand der Dichtlippe 15 liegt bei etwa 0,5 mm, während die Dichtlippe 15 über die Unterseite der Membranfläche 10, durch den Abstand 17 in Figur 2 angedeutet, etwa 1 bis 1,5 mm übersteht. Der Durchmesser 18 des Stopfens am Ende der umlaufenden Dichtlippe 15 ist etwa 1 mm größer als der Inenendurchmesser der Patrone 2. Beim Einsetzen des Stopfens in die Patrone 2 wird die umlaufende Dichtlippe 15 radial nach innen in Richtung des Pfeils 19 gedrückt und damit ein Moment auf die Membranfläche 10 ausgeübt, so daß deren Wölbung zur Innenseite des becherförmigen Stopfens 6 hin verstärkt wird, wie durch die unterbrochene Linie 20 in Figur 2 dargestellt ist. Diese Vorspannung bewirkt einerseits eine große Auslenkung der Membranfläche 10 zum Drehkolben 4 hin und andererseits wird über die Membranfläche 10 eine Kraft auf die umlaufende Dichtlippe 15 zu der Innenwand der Patrone 2 hin ausgeübt und damit die Dichtwirkung der Dichtlippe gegenüber der Innenwandung der Patrone 2 verstärkt.

In Figur 3 ist in der nach rechts verschobenen Position des Stopfens 6 eine Stellung gezeigt, in die der Stopfen 6 nach dem Befüllen einer Patrone 2 mit viskosem Dentalmaterial verschoben ist. In dieser Stellung stößt der Stopfen 6 mit der freien Stirnseite der Seitenwände 11 gegen eine Schulter 21 an. Von dieser Stellung ausgehend wird der Drehkolben 4, der ein abgerundetes Ende 22 besitzt, und zwar mit einem Radius, der dem halben Durchmesser des Drehkolbens 4 entspricht, durch Drehung um die Achse 16 in Richtung des Pfeils 24 verschoben. Mit dieser Verschiebung wird die Membranfläche 10 in umgekehrter Richtung gewölbt, wie dies in der in Figur 3 rechts gezeigten Position des Stopfens 9 dargestellt ist. Die Bodenfläche 10 wölbt sich entsprechend der Rundung des Endes 22 des Drehkolbens 4, so daß auch bei einer großen Kraftbeaufschlagung keine Beschädigung der Membranfläche 10 auftritt. Gleichzeitig wird in dieser an dem Ende 22 des Drehkolbens 4 anliegenden Stellung ein Moment auf die umlaufende Dichtlippe 15 ausgeübt, die radial zu der Achse 16 nach außen gegen die Innenwand der Patrone 2 gedrückt wird. Nachdem eine gewünschte Menge an Material aus der Patrone 2 über die Austragsöffnung 23 abgegeben ist, wird der Drehkolben 4 in die entgegengesetzte Richtung gedreht und damit der Druck von der Membranfläche 10 genommen; der Becherboden bzw. die Membranfläche 10 kehrt dann wieder in ihre vorgespannte Ausgangsstellung, die auf der rechten Seite der Figur 3 gezeigt ist, zurück. Mit dem Zurückklappen oder -schwenken der Membranfläche 10 wird bereits in der Anfangsphase in der Patrone ein auf das Material einwirkender Unterdruck erzeugt, der das Material, das sich noch in der Austragsöffnung 23 der Austrittsdüse 9 befindet, in die Patrone 2 hineinzieht. Ein Nachlaufen oder ein Nachtropfen tritt dadurch nicht auf.

Die Spritze bzw. Patrone 2, die mit dem dargestellten Stopfen 6 auf der einen Seite verschlossen wird, hat den Vorteil, daß sie von vorne, d.h. von dem austragsseitigen Ende der Patrone 2 aus, herstellerseitig mit Material befüllt werden kann. Hierzu wird zunächst in die leere Patrone 2 der Stopfen 6 eingesetzt, der sich dichtend mit der umlaufenden Dichtlippe 15 an die Innenwand der Patrone 2 anlegt. Anschließend wird die Patrone in eine Befüllstation eingesetzt und der Werkstoff in die Patrone 2 eingedrückt, wobei sich der Stopfen 6 zum hinteren Ende der Patrone in Richtung des Pfeils 25 in Figur 2 verschiebt, bis der Stopfen 6 an der Schulter 21 anliegt. Bei diesem Befüllvorgang treten keine Lufteinschlüsse im Bereich des Stopfens 6 auf, da vor dem Stopfen befindliche Luft bereits in der Anfangsphase des Befüllvorgangs mit Material aus der Patrone 2 austritt. Anschließend wird die befüllte Patrone 2 verschlossen. Beim Befüllen der Patrone 2 wird durch das auf die Bodenfläche 10 des Stopfens 6 drückende Material die umlaufende Dichtlippe 15 entlastet, so daß sich der Stopfen 6 sehr leicht entlang der Innenwand der Patrone 2 verschieben läßt.

Der Stopfen 6 ist aus einem Polyethylen hergestellt, das einen Vorteil dahingehend bietet, daß es einerseits ausreichend flexibel ist, andererseits eine ausreichende Festigkeit auch im Bereich dünner Stellen besitzt.

## Patentansprüche

1. Spritze zum dosierten Abgeben von pastösen Werkstoffen mit einer den Werkstoff aufnehmenden Patrone (2), in die von deren einem Ende aus ein Drehkolben (4) eintaucht, wobei der Drehkolben an seinem Eintauchende gegen einen Stopfen (6), der ein scheibenförmiges Flächenteil aufweist, das den Innenquerschnitt der Patrone dichtend verschließt, anliegt, dadurch gekennzeichnet, daß das scheibenförmige Flächenteil des Stopfens (6) mindestens teilweise als Membranfläche (10) ausgebildet ist, die im entlasteten Zustand eine stabile Lage bildend gegen die Eintauchrichtung des Drehkolbens (4) so vorgespannt ist, daß sie nach einer Verschiebung durch den Drehkolben (4) im entlasteten Zustand in diese stabile Lage reversibel zurückkehrt, und daß die Membranfläche (10) aus Kunststoff gebildet ist.

2. Spritze nach Anspruch 1, dadurch gekennzeichnet, daß der Stopfen (6) becherförmig und dessen Bodenfläche als elastisch verformbare Membranfläche (10) ausgebildet ist.

3. Spritze nach Anspruch 1 oder 2, dadurch gekennzeichnet, daß die Membranfläche (10) gegen die Eintauchrichtung des Drehkolbens (4) in Form einer Wölbung vorgespannt ist.

4. Spritze nach einem der Ansprüche 1 bis 3, dadurch gekennzeichnet, daß der Stopfen (6) an seiner Außenseite im Bereich seiner Anlage an die Innenwand der Patrone (2) eine Dichtlippe (15) aufweist.

5. Spritze nach Anspruch 4, dadurch gekennzeichnet, daß die Dichtlippe (15) umlaufend ausgebildet ist, die über die Membranfläche (10) nach unten und über die Seitenwände (11) nach außen vorsteht.

6. Spritze nach einem der Ansprüche 2 bis 5, dadurch gekennzeichnet, daß auf der Innenseite des becherförmigen Stopfens (6) im Übergangsbereich zwischen der Becherwand (11) und der Membranfläche (10) eine zumindest teilweise umlaufende Nut (13) ausgebildet ist.

7. Spritze nach Anspruch 5 und 6, dadurch gekennzeichnet, daß die umlaufende Dichtlippe (15) gegen die Innenwand der Patrone (2) unter Spannung anliegend ein gegen die Eintauchrichtung des Drehkolbens (4) gerichetes Moment auf die Membranfläche (10) ausübt.

8. Spritze nach einem der Ansprüche 2 bis 7, dadurch gekennzeichnet, daß die Wand (11) auf der Außenseite des becherförmigen Stopfens (6) radial umlaufende Rillen und Nuten und/oder Vorsprünge (14) aufweist.

9. Spritze nach einem der Ansprüche 2 bis 8, dadurch gekennzeichnet, daß sich der Durchmesser der Seitenwände (11) des Stopfens (6) zu deren freiem Ende hin vergrößert.

10. Spritze nach einem der Ansprüche 1 bis 8, dadurch gekennzeichnet, daß das Ende (22) des Drehkolbens (4) gewölbt ist.

11. Spritze nach Anspruch 10, dadurch gekennzeichnet, daß die Wölbung des Endes (22) des Drehkolbens (4) entsprechend der maximalen Auslenkung der Membranfläche (10) des Stopfens (6) in Vorschubrichtung des Drehkolbens (4) ausgebildet ist.

12. Spritze nach einem der Ansprüche 1 bis 11, dadurch gekennzeichnet, daß die Patrone (2) zum Material hin auf der einen Seite dichtend abschließt.

13. Spritze nach einem der Ansprüche 1 bis 12, dadurch gekennzeichnet, daß der Stopfen (6) an dem Eintauchende des Drehkolbens (4) gegen einen Anschlag (14), der an der Patrone (2) gebildet ist, anliegt.

14. Spritze nach einem der Ansprüche 1 bis 13, dadurch gekennzeichnet, daß die Membranfläche (10) eine Dicke zwischen 0,2 und 0,6 mm aufweist.

15. Spritze nach Anspruch 5, dadurch gekennzeichnet, daß die umlaufende Dichtlippe (15) 0,2 - 0,7 mm über die Seitenwände (11) nach außen vorsteht.

16. Spritze nach Anspruch 5, dadurch gekennzeichnet, daß die umlaufende Dichtlippe (15) 0,3 - 0,5 mm über die Membranfläche (10) nach unten vorsteht.

17. Spritze nach Anspruch 6, dadurch gekennzeichnet, daß die umlaufende Nut (13) einen Radius von etwa 0,5 mm aufweist.

## Claims

1. Syringe for the controlled discharge of paste-like materials having a cartridge (2) for holding the materials into which a rotary piston (4) plunges from the one end thereof, whith the rotary piston with its one plunging end abutting against a stopper (6) having a disc-shaped partial surface which sealingly closes the inside cross section of the cartridge, characterized in that the disc-shaped partial surface of the stopper (6) is configured at least partly as a membrane surface (10) which, in a depressurized state, is prestressed assuming a stable position with respect to the plunging direction of the rotary piston (4) so that after a displacement by the rotary piston (4), the disc-shaped partial surface returns reversibly to the stable position in the depressurized state and in that the membrane surface (10) is formed from plastic material.

2. Syringe as claimed in claim 1, characterized in that the stopper (6) is cup-shaped and has a base surface configured as an elastically deformable membrane surface (10).

3. Syringe as claimed in one of claims 1 or 2, characterized in that the membrane surface (10) is prestressed in the form of a curvature opposite to the plunging direction of the rotary piston (4).

4. Syringe as claimed in one of claims 1 to 3, characterized in that the stopper (6) comprises a sealing lip (15) on its outside portion in the region of contact with an inside wall of the cartridge (2).

5. Syringe as claimed in claim 4, characterized in that the sealing lip (15) is configured in an encircling manner, projecting downward beyond the membrane surface (10) and outward beyond the side wall (11).

6. Syringe as claimed in one of claims 2 to 5, characterized in that on an inside of the cup-shaped stopper (6), in a transitional region between a side wall (11) of the cup and the membrane surface (10), there is formed an at least partly encircling groove (13).

7. Syringe as claimed in claim 5 and claim 6, characterized in that the encircling sealing lip (15), which is in contact under tension against the inside wall of the cartridge (2), exerts a moment on the membrane surface (10) which is directed opposite to the plunging direction of the rotary piston (4).

8. Syringe as claimed in one of claims 2 to 7, characterized in that the wall (11) on an outside of the cup-shaped stopper (6) comprises radially-encircling channels and grooves and/or projections (14).

9. Syringe as claimed in one of claims 2 to 8, characterized in that the diameter of the side walls (11) of the stopper (6) increases toward their free ends.

10. Syringe as claimed in one of the claims 1 to 8, characterized in that the end (22) of the rotary piston (4) is curved.

11. Syringe as claimed in claim 10, characterized in that the curvature of the end (22) of the rotary piston (4) is configured according to the maximum travel of the membrane surface (10) of the stopper (6) in a direction of forward motion of the rotary piston (4).

12. Syringe as claimed in one of claims 1 to 11, characterized in that the cartridge (2) forms a tight seal toward the material on the one side.

13. Syringe as claimed in one of claims 1 to 12, characterized in that the stopper (6) on the plunging end of the rotary piston (4) abuts against a stop (14) formed on the cartridge (2).

14. Syringe as claimed in one of claims 1 to 13, characterized in that the membrane surface (10) has a thickness between 0.2 and 0.6 mm.

15. Syringe as claimed in claim 5, characterized in that the encircling sealing lip (15) projects outward by a distance in the range of 0.2 to 0.7 mm beyond the side walls (11).

16. Syringe as claimed in claim 5, characterized in that the encircling sealing lip (15) projects downward by a distance in the range of 0.3 to 0.5 mm beyond the membrane surface (10).

17. Syringe as claimed in claim 6, characterized in that the encircling groove (13) has a radius of about 0,5 mm.

## Revendications

1. Seringue de dosage de substances pasteuses avec une cartouche (2) qui contient la substance, cartouche dans laquelle plonge un piston rotatif (4) à partir de l'une de ses extrémités, le piston rotatif s'appuyant, à son extrémité immergée, contre un bouchon (6) qui présente une partie plane en forme de disque qui ferme en l'étanchant la section intérieure de la cartouche, caractérisée en ce que la partie plane en forme de disque du bouchon (6) est configurée au moins partiellement comme surface à membrane (10) qui, à l'état détendu, est précontrainte contre le sens d'immersion du piston rotatif (4) en formant une position stable de telle manière qu'après une translation par le piston rotatif (4) elle retourne de manière réversible dans cette position stable à l'état détendu et que la surface à membrane (10) est formée en matière plastique.

2. Seringue selon la revendication 1, caractérisée en ce que le bouchon (6) est configuré en forme de godet et sa surface de fond est configurée comme surface à membrane (10) déformable élastiquement.

3. Seringue selon la revendication 1 ou 2, caractérisée en ce que la surface à membrane (10) est précontrainte en forme de voussure contre le sens d'immersion du piston rotatif (4).

4. Seringue selon l'une des revendications 1 à 3, caractérisée en ce que le bouchon (6) présente une lèvre d'étanchéité (15) sur sa face extérieure, dans la zone de son appui sur la paroi intérieure de la cartouche (2).

5. Seringue selon la revendication 4, caractérisée en ce que la lèvre d'étanchéité (15) est configurée périphérique en faisant saillie au-dessus de la surface à membrane (10) vers le bas et au-dessus des parois latérales (11) vers l'extérieur.

6. Seringue selon l'une des revendications 2 à 5, caractérisée en ce que, sur la face intérieure du bouchon en forme de godet (6), une gorge (13) au moins partiellement périphérique est configurée dans la zone de transition entre la paroi du godet (11) et la surface à membrane (10).

7. Seringue selon les revendications 5 et 6, caractérisée en ce que la lèvre d'étanchéité périphérique (15), en s'appuyant contre la paroi intérieure de la cartouche (2) en étant sous tension, exerce un moment sur la surface à membrane (10) qui est orienté contre le sens d'immersion du piston rotatif (4).

8. Seringue selon l'une des revendications 2 à 7, caractérisée en ce que la paroi (11) présente, sur la face extérieure du bouchon en forme de godet (6), des rainures et gorges et/ou saillies (14) périphériques dans le sens radial.

9. Seringue selon l'une des revendications 2 à 8, caractérisée en ce que le diamètre des parois latérales (11) du bouchon (6) augmente en direction de son extrémité libre.

10. Seringue selon l'une des revendications 1 à 8, caractérisée en ce que l'extrémité (22) du piston rotatif (4) est voûtée.

11. Seringue selon la revendication 10, caractérisée en ce que la voussure de l'extrémité (22) du piston rotatif (4) est configurée selon l'excursion maximale de la surface à membrane (10) du bouchon (6) dans le sens de l'avancement du piston rotatif (4).

12. Seringue selon l'une des revendications 1 à 11, caractérisée en ce que la cartouche (2) se termine en étanchant vers la substance de l'un des côtés.

13. Seringue selon l'une des revendications 1 à 12, caractérisée en ce que le bouchon (6) adhère à l'extrémité d'immersion du piston rotatif (4) contre une butée (14) qui est formée sur la cartouche (2).

14. Seringue selon l'une des revendications 1 à 13, caractérisée en ce que la surface à membrane (10) présente une épaisseur entre 0,2 et 0,6 mm.

15. Seringue selon la revendication 5, caractérisée en ce que la lèvre d'étanchéité périphérique (15) fait saillie vers l'extérieur au-dessus des parois latérales (11) de 0,2 à 0,7 mm.

16. Seringue selon la revendication 5, caractérisée en ce que la lèvre d'étanchéité périphérique (15) fait saillie vers le bas au-dessus de la surface à membrane (10) de 0,3 à 0,5 mm.

17. Seringue selon la revendication 6, caractérisée en ce que la gorge périphérique (13) présente un rayon d'environ 0,5 mm.
